Europäisches Patentamt

European Patent Office

Office européen des brevets

(11) Publication number: **0 107 877**
**B1**

## (12) EUROPEAN PATENT SPECIFICATION

(45) Date of publication of patent specification: **07.02.90**

(51) Int. Cl.⁵: **C 07 C 2/76, B 01 J 29/04**

(21) Application number: **83201422.9**

(22) Date of filing: **04.10.83**

(54) **Process for the preparation of an aromatic hydrocarbon mixture.**

(30) Priority: **28.10.82 NL 8204168**
**30.11.82 NL 8204631**

(43) Date of publication of application:
**09.05.84 Bulletin 84/19**

(45) Publication of the grant of the patent:
**07.02.90 Bulletin 90/06**

(84) Designated Contracting States:
**BE DE FR GB IT NL**

(56) References cited:
**EP-A-0 021 475**
**EP-A-0 024 147**
**EP-A-0 089 795**
**DE-A-2 755 770**
**FR-A-2 374 283**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Kieffer, Eduard Philip**
**Badhuisweg 3**
**NL-1031 CM Amsterdam (NL)**

(74) Representative: **Aalbers, Onno et al**
**P.O. Box 302**
**NL-2501 CH The Hague (NL)**

Courier Press, Leamington Spa, England.

**Description**

The present invention relates to a process for the preparation of an aromatic hydrocarbon mixture, in which one or more paraffins with two, three or four carbon atoms per molecule or aliphatic hydrocarbon mixtures consisting more than 50%w of said paraffins are contacted with a catalyst comprising gallium supported on a carrier and a crystalline metal silicate which metal silicate

a) has been prepared by crystallization stating from an aqueous mixture which contains one or more compounds of an alkali metal, one or more organic nitrogen compounds which contain an organic cation or from which anorganic cation is formed during the preparation of the silicate, one or more silicon compounds, one or more compounds of a trivalent metal Y chose from the group formed by aluminium, iron, cobalt and chromium and, if desired, one or more gallium compounds in such quantities that in the formula which represents the composition of the silicate expressed in moles of the oxides, the $SiO_2/(Y_2O_3 + Ca_2O_3)$ molar ratio is 10—500 and the $Ca_2O_3/Y_2O_3$ molar ratio is lower than 1, and

b) after one hour's calcination in air at 500°C has an X-ray power diffraction pattern in which the strongest lines are the four lines mentioned in Table A,

TABLE A

| $d(Å)$ |
| --- |
| $11.1 \pm 0.2$ |
| $10.0 \pm 0.2$ |
| $3.84 \pm 0.07$ |
| $3.72 \pm 0.06$ |

in which catalyst the quantity of gallium supported on a carrier amounts to 1—10%w, calculated on the quantity of crystalline metal silicate present in the catalyst and which catalyst has been subjected at least once to a two-step treatment comprising a step in which the catalyst is contacted at a temperature of 350—700°C with a hydrocarbon or a mixture of hydrocarbons until in less than 5 hours 0.1—5%w of coke has been deposited on the catalyst, followed by a second step in which the coke-loaded catalyst is contacted at a temperature of 350—700°C with an oxygen-containing gas until more than 50%w of the coke present on the catalyst has been removed.

By subjecting the catalysts at least once to the above two-step treatment, their performance in the conversion of said paraffins into aromatics is much improved. The treatment results in a substantial increase of the $C_5^+$ selectivity. In addition it leads to enhancement of the activity and $H_2$ selectivity of the catalysts. The $H_2$ selectivity increase represents an advantage since in view of the growing demand for hydrogen for a variety of purposes it is important that in the conversion as much as possible of the hydrogen becomes available as molecular hydrogen and not in the form of hydrogen-rich by-products, such as methane.

The liquid hydrocarbon mixtures obtained in the process according to the invention boil substantially in the gasoline range and have a very high octane number. They are therefore excellently suitable for use as motor gasoline or as mixing components for motor gasolines.

A carrier for the gallium that may suitably be used is the crystalline silicate of the special structure described above which silicate is present in the catalysts and onto which silicate the gallium has been deposited, for instance by impregnation or ion exchange. In the catalysts the gallium occurring may be partly or wholly deposited on a conventional carrier, such as silica, the gallium-loaded carrier being present in the catalyst in admixture with a crystalline silicate of the special structure.

The quantity of crystalline metal silicate, calculated on the sum of the quantity of crystalline metal silicate present in the catalyst and the quantity of other material used as carrier for the gallium which may be present in the catalyst, is preferably at least 20%w.

In the process according to the invention the starting material comprises paraffins having two, three or four carbon atoms per molecule or an aliphatic hydrocarbon mixture which consists more than 50%w of said paraffins. The paraffins with two, three or four carbon atoms per molecule which should constitute more than 50%w of the feed are ethane, propane, n-butane and isobutane. If the starting material is an aliphatic hydrocarbon mixture which, in addition to the paraffins mentioned, contains other aliphatic hydrocarbons as well, this mixture may contain, inter alia, methane, ethene, propene, butene, isobutene, butadiene and paraffins and olefins with five or more carbon atoms per molecule. In the process according to the invention the starting material preferred is a feed which consists more than 75%w, and in particular substantially completely, of one or more paraffins having three or four carbon atoms per molecule. A feedstock which is very suitable for use in the process is a mixture of paraffins with three or four carbon atoms per molecule obtained as a by-product in the production of mineral oil.

The process according to the invention is preferably carried out a temperature of 350—700°C and in

2

particular of 450—650°C, a pressure of 1—20 bar and in particular of 1—10 bar and a space velocity of $0.1$—$10$ $kg.kg^{-1}.hour^{-1}$ and in particular of $0.05$—$5$ $kg.kg^{-1}.hour^{-1}$.

In the process according to the invention the feed is contacted with a catalyst containing a crystalline metal silicate which is defined, among other things, by the X-ray powder diffraction pattern which the silicate shows after one hour's calcination in air at 500°C. In this pattern the strongest lines should be the four lines mentioned in Table A. The complete X-ray powder diffraction pattern of a typical example of the present crystalline metal silicates after one hour's calcination in air at 500°C is given in Table B.

TABLE B

| d(Å) | Rel. int. | d(Å) | Rel. int. |
|---|---|---|---|
| 11.1 | 100 | 3.84 (D) | 57 |
| 10.0 (D) | 70 | 3.72 (D) | 31 |
| 8.93 | 1 | 3.63 | 16 |
| 7.99 | 1 | 3.47 | < 1 |
| 7.42 | 2 | 3.43 | 5 |
| 6.68 | 7 | 3.34 | 2 |
| 6.35 | 11 | 3.30 | 5 |
| 5.97 | 17 | 3.25 | 1 |
| 5.70 | 7 | 3.05 | 8 |
| 5.56 | 10 | 2.98 | 11 |
| 5.35 | 2 | 2.96 | 3 |
| 4.98 (D) | 6 | 2.86 | 2 |
| 4.60 | 4 | 2.73 | 2 |
| 4.35 | 5 | 2.60 | 2 |
| 4.25 | 7 | 2.48 | 3 |
| 4.07 | 2 | 2.40 | 2 |
| 4.00 | 4 | | |

(D) = doublet

The catalysts used in the process according to the invention are catalysts comprising the prescribed percentage of gallium supported on a carrier and in addition a crystalline metal silicate of the special structure which as a $SiO_2/(Y_2O_3 + Ga_2O_3)$ molar ratio of 10—500. A carrier for the gallium that may suitably be used in the crystalline metal silicate. The gallium is preferably deposited on the crystalline metal silicate by impregnation or ion exchange starting from an aqueous solution of a gallium compound, such as gallium nitrate. Very suitable catalysts for carrying out the present conversion may be prepared by depositing the gallium on a conventional carrier, such as silica and mixing the gallium-loaded carrier with the crystalline metal silicate. Mixtures of gallium supported on silica as the carrier and gallium supported on the crystalline metal silicate as the carrier are also suitable for use as catalyst in the present conversion. In the process according to the invention preference is given to the catalysts in which the carrier for the gallium is exclusively the crystalline metal silicate and in which the gallium has been deposited on the crystalline metal silicate by impregnation or ion exchange, as well as to catalysts in which the carrier for the gallium is exclusively an amorphous material, the gallium-loaded carrier is present in the catalyst in admixture with the crstyalline metal silicate.

The crystalline metal silicate which is present in the catalysts as the carrier for the gallium and/or as a mixing component for a gallium-loaded carrier has a $SiO_2/(Y_2O_3 + Ga_2O_3)$ molar ratio of 10—500. It is pointed out that in determining the $SiO_2/(Y_2O_3 + Ga_2O_3)$ molar ratio only the quantity of gallium should be taken into account which may have been incorporated in the silicate during the synthesis of the silicate by crystallization from an aqueous solution containing one or more gallium compounds. Neither gallium which has been deposited on the ready crystalline metal silicate after the synthesis, for instance by impregnation or ion exchange, nor gallium which is present in the catalyst supported on a carrier plays a role in the determination of the $SiO_2/(Y_2O_3 + Ga_2O_3)$ molar ratio of the crystalline metal silicate.

In the process according to the invention preference is given to the use of a catalyst comprising a crystalline metal silicate with a $SiO_2/(Y_2O_3 + Ga_2O_3)$ molar ratio of 25—250.

As regards the number of times that the two-step treatment should be carried out in order to obtain a catalyst with optimum performance in the present conversion, it may be remarked, that in general, the performance of the present catalysts can be brought to an optimum level by subjecting them at most three times to the two-step treatment.

Further investigation has shown that, a preceding calcination at a temperature between 600 and 1000°C, is detrimental to the catalyst performance in the case of the present catalysts.

In the first step of the two-step treatment basically any hydrocarbon or mixture of hydrocarbons may be used, provided that at a temperature of 350—700°C and in a period of less than 5 hours a coke deposition on the catalyst of 0.1—5%w can thereby be realized. For this purpose the hydrocarbon or the mixture of hydrocarbons used as the feed in the process according to the invention is very suitable.

The oxgyen-containing gas used in the second step of the two-step treatment for the removal of more than 50%w of the coke present on the catalyst may very suitably be air. Preferably more than 75%w of the coke present on the catalyst and in particular substantially all the coke present on the catalyst is removed in the second step of the two-step treatment.

The preparation of the crystalline metal silicates used in the process according to the invention is carried out starting from an aqueous mixture containing the following compounds: one or more compounds of an alkali metal (M), one or more organic nitrogen compounds (RN) which contain an organic cation of from which an organic cation is formed during the preparation of the silicate, one or more silicon compounds, one or more compounds of a trivalent metal Y and, if desired one or more gallium compounds. The preparation is suitably carried out by maintaining the mixture at an elevated temperature until the silicate has formed and subsequently separating the silicate crystals from the mother liquor and washing, drying and calcining the crystals. In the aqueous mixture from which the silicates are prepared the various compounds should suitably be present in the following molar ratios expressed — with the exception of the organic nitrogen compounds — in moles of the oxides:

$$M_2O:SiO_2 = 0.01—0.35,$$

$$RN:SiO_2 = 0.20—1.0,$$

$$SiO_2:(Y_2O_3 + Ga_2O_3) = 10—750,$$

$$Ga_2O_3:Y_2O_3 <1, \text{ and}$$

$$H_2O:SiO_2 = 5—65.$$

In the preparation of the silicates the base mixture may very suitably be a mixture containing a quaternary ammonium compound as the organic nitrogen compound a sodium compound as the alkali metal compound and amorphous silica as the silicon compound.

In the process according to the invention preference is given to the use of crystalline metal silicates which have been prepared by crystallization from an aqueous mixture which contains no gallium compounds and which contains at least one or more aluminium compounds as the compounds of a trivalent metal Y.

The silicates prepared as described hereinbefore contain alkali metal ions. By using suitable exchange methods these may be replaced by other cations, such as hydrogen ions or ammonium ions. The crystalline metal silicates used in the process according to the invention preferably have an alkali metal content of less than 0.05%w. In the process according to the invention the compositions comprising gallium supported on a carrier as well as a crystalline metal silicate or a special structure may be used per se or combined with a binder material, such as kaolin or bentonite.

The invention is now elucidated with the aid of the following example.

## Example

Three crystalline aluminium silicates (silicates 1—3) were prepared by heating mixtures of NaOH, amorphous silica, $(CH_3H_7)_4NOH$ and $NaAlO_2$ in water, in an autoclave under autogenous pressure, at 150°C for 24 hours. After cooling of the reaction mixtures the silicates formed were filtered off, washed with water until the pH of the wash water was about 8 and dried at 120°C. After one hour's calcination in air at 500°C silicates 1—3 had the following properties

a) an X-ray powder diffraction pattern substantially corresponding with that mentioned in Table B, and

b) a $SiO_2/Al_2O_3$ molar ratio as mentioned in Table C.

TABLE C

| Silicate No. | $SiO_2/Al_2O_3$ molar ratio |
|---|---|
| 1 | 100 |
| 2 | 100 |
| 3 | 170 |

The molar compositions of the aqueous mixtures from which silicates 1—3 were prepared may be rendered as follows: x $Na_2O$.y $(C_3H_7)_4NOH$.z $Al_2O_3$.25 $SiO_2$.450 $H_2O$ wherein x, y and z have the values given in Table D.

TABLE D

| Silicate No. | x | y | z |
|---|---|---|---|
| 1 | 0.33 | 9 | 0.33 |
| 2 | 1 | 3 | 0.2 |
| 3 | 1 | 9 | 0.1 |

From silicates 1—3 were prepared silicates I—III, respectively, by boiling silicates 1—3 with a 1.0 molar $NH_4NO_3$ soluton, washing with water, boiling again with a 1.0 molar $NH_4NO_3$ solution and washing, drying at 120°C and calcination at 500°C.

Catalysts I—III were prepared from silicates I—III as follows:

Catalyst I

This catalyst was prepared by impregnating silicate I with an aqueous solution of gallium nitrate. Catalyst I contained 2%w gallium.

Catalyst II

This catalyst was prepared by ion exchange of silicate II using an aqueous solution of gallium nitrate. Catalyst II contained 3.2%w gallium.

Catalyst III

This catalyst was prepared by mixing silicate III in a weight ratio of 1:1 with a composition which contained 2%w gallium on silica and had been obtained by impregnation of silica with an aqueous solution of gallium nitrate. Catalyst III contained 1%w gallium.

Samples of catalyst I—III were subjected once or several times to a two-step treatment comprising a step in which the catalyst was contacted with n-butane for 30 minutes at a temperature of 600°C, a pressure of 1.5 bar and a space velocity of 8 $g.g^{-1}.h^{-1}$ and in which a quantity of coke ranging from 0.2 to 0.8%w was deposited on the catalyst, followed by a second step in which the catalyst was contacted with air for 1 hour at a temperature of 500°C and a pressure of 1.5 bar in which 95—99%w of the coke present on the catalyst was removed. From catalysts I—III were thus produced catalysts IA, IB, IIA, IIIA, respectively. In addition a sample of catalyst III was first contacted with air for 1 hour at 700°C and then subjected twice to the two-step treatment described hereinbefore. Thus catalyst IIIB was obtained from catalyst III.

Catalysts I—IB, II, IIA and III—IIIB were tested in eight experiments (Experiments 1—8) in the preparation of $C_5^+$ aromatic hydrocarbon mixtures starting from n-butane. The experiments were carried out in a reactor containing a fixed catalyst bed. All the experiments were carried out at a temperature of 550°C, a pressure of 1.5 bar and a space velocity of 2 $kg.kg^{-1}.hour^{-1}$. The results of the experiments are listed in Table E. Table E also indicates how many times each of the catalysts has been subjected to the two-step treatment as well as the quantities of coke deposited on each catalyst in the first step of the two-step treatment.

Of the experiments mentioned in Table E Experiments 2, 3, 5 and 7 are experiments according to the invention. These experiments were carried out using catalysts comprising gallium supported on a carrier as well as a crystalline metal silicate of a special structure, which catalysts had been subjected to at most three two-step treatments according to the invention. These catalysts show high activity and $H_2$ selectivity as well as acceptable $C_5^+$ selectivity. Comparison of the results of Experiments 1—3 shows that the catalyst performance is much enhanced if the catalyst is subjected to the two-step treatment once, but that the performance shows no marked further improvement when the two-step treatment is repeated three times.

Experiments 1, 4, 6 and 8 fall outside the scope of the invention. They have been included in the patent application for comparison. Experiments 1, 4 and 6 were carried out using catalysts comprising gallium supported on a carrier and a crystalline metal silicate of a special structure, but these catalysts had not been subjected to a two-step treatment according to the invention. Experiment 8 was carried out using a catalyst comprising gallium supported on a carrier and a crystalline metal silicate of a special structure, which catalyst had been subjected twice to a two-step treatment according to the invention, but this catalyst had been subjected to calcination at 700°C preceding the repeated two-step treatment.

TABLE E

| Experiment No. | 1 | 2 | 3 | 4 | 5 | 6 | 7 | 8 |
|---|---|---|---|---|---|---|---|---|
| Catalyst No. | I | IA | IB | II | IIA | III | IIIA | IIIB |
| Number of times that catalyst was subjected to two-step treatment | — | 1 | 3 | — | 3 | — | 2 | 2* |
| Quantity of coke deposited on catalyst in first step of two step treatment, %w | — | 0.2 | 0.2—0.8 | — | 0.8—0.9 | — | 0.7—0.8 | 0.7—0.8 |
| Conversion, %w | 51 | 71 | 70 | 97 | 98 | 91 | 92 | 86 |
| Product selectivity, %w on converted material | | | | | | | | |
| $H_2$ | 3.5 | 5.8 | 5.5 | 4.5 | 5.3 | 2.3 | 4.9 | 3.5 |
| $C_1$—$C_3$ | 74.2 | 58.4 | 52.4 | 68.8 | 54.0 | 78.0 | 62.8 | 69.2 |
| iso—$C_4$° | 4.7 | 3.3 | 3.4 | 0.9 | 0.3 | 2.0 | 1.3 | 1.4 |
| $\Sigma\, C_4^=$ | 3.4 | 2.2 | 4.9 | 0.6 | 0.5 | 1.4 | 0.9 | 1.0 |
| $C_5^+$ | 14.2 | 30.3 | 33.8 | 25.2 | 39.9 | 16.4 | 30.1 | 24.9 |

\* After preceding treatment with air at 700°C

**Claims**

1. A process for the preparation of an aromatic hydrocarbon mixture, characterized in that paraffins with two, three or four carbon atoms per molecule or aliphatic hydrocarbon mixtures consisting more than 50%w of said paraffins are contacted with a catalyst comprising gallium supported on a carrier and a crystalline metal silicate which

a) has been prepared by crystallization stating from an aqueous mixture which contains one or more compounds of an alkali metal, one or more organic nitrogen compounds which contain an organic cation or from which an organic cation is formed during the preparation of the silicate, one or more silicon compounds, one or more compounds of a trivalent metal Y chose from the group formed by aluminium, iron, cobalt and chromium and, if desired, one or more gallium compounds in such quantities that in the formula which represents the composition of the silicate expressed in moles of the oxides, the $SiO_2/(Y_2O_3 + Ca_2O_3)$ molar ratio is 10—500 and the $Ca_2O_3/Y_2O_3$ molar ratio is lower than 1, and

b) after one hour's calcination in air at 500°C has an X-ray powder diffraction pattern in which the strongest lines are the four lines mentioned in Table A,

TABLE A

| d(Å) |
|---|
| 11.1 ± 0.2 |
| 10.0 ± 0.2 |
| 3.84 ± 0.07 |
| 3.72 ± 0.06 |

6

in which catalyst the quantity of gallium supported on a carrier amounts to 1—10%w, calculated on the quantity of crystalline metal silicate present in the catalyst and which catalyst has been subjected at least once to a two-step treatment comprising a step in which the catalyst is contacted at a temperature of 350—700°C with a hydrocarbon or a mixture of hydrocarbons until in less than 5 hours 0.1—5%w of coke has been deposited on the catalyst, followed by a second step in which the coke-loaded catalyst is contacted at a temperature of 350—700°C with an oxygen-containing gas until more than 50%w of the coke present on the catalyst has been removed.

2. A process as claimed in claim 1, characterized in that it is applied to a feedstock more than 75%w of which consists of one or more paraffins having three or four carbon atoms per molecule.

3. A process as claimed in claim 1 or 2, characterized in that it is carried out at a temperature of 350—700°C, a pressure of 1—20 bar and a spare velocity of 0.1—10 $kg.kg^{-1}.h^{-1}$.

4. A process as claimed in any one of claims 1—3, characterized in that the caalyst is subjected to the two-step treatment at most three times.

5. A process as claimed in any one of claims 1—4, characterized in that the quantity of crystalline metal silicate, calculated on the sum of the quantity of crystalline metal silicate present in the catalyst and the quantity of other material used as carrier for the gallium which may be present in the catalyst, is at least 20%w.

6. A process as claimed in any one of claims 1—5, characterized in that the gallium present in the catalyst is supported on the crystalline metal silicate as carrier and that the gallium has been deposited on the crystalline metal silicate by impregnation or ion exchange.

7. A process as claimed in any one of claims 1—5, characterized in that the gallium present in the catalyst is supported on an amorphous carrier material.

8. A process as claimed in any one of claims 1—7, characterized in that the feedstock is used to achieve the desired level of coke deposition in the first step of the two-step treatment.

9. A process as claimed in any one of claims 1—8, characterized in that the air is used as the oxygen-containing gas in the second step of the two-step treatment.

10. A process as claimed in any one of claims 1—9, characterized in that the second step of the two-step treatment more than 75%w of the coke present on the catalyst is removed.

## Patentansprüche

1. Ein Verfahren zur Herstellung einer aromatischen Kohlenwasserstoffmischung, dadurch gekennzeichnet, daß Paraffine mit 2, 3 oder 4 Kohlenstoffatomen je Molekül oder aliphatische Kohlenwasserstoffmischungen, welches mehr als 50 Gewichtsprozent besagter Paraffine enthalten, mit einem Katalysator kontaktiert werden, welcher Gallium auf einem Träger aufgebracht und ein kristallines Metallsilikat enthält, welches

a) durch Kristallisation aus einer wäßrigen Ausgangsmischung hergestellt worden ist, welche eine oder mehrere Verbindungen eines Alkalimetalls, eine oder mehrere organische Stickstoffverbindungen, welche ein organisches Kation aufweisen oder aus denen sich während der Herstellung des Silikats ein organisches Kation bildet, eine oder mehrere Siliciumverbindungen, ein oder mehrere Verbindungen eines dreiwertigen Metalles Y, ausgewählt aus der Gruppe Aluminium, Eisen, Kobalt und Chrom, und gegebenenfalls eine oder mehrere Galliumverbindungen in solchen Mengen enthält, daß in der die Zusammensetzung des Silikats wiedergebenden Formel, ausgedrückt in Molen der Oxide, das Molverhältnis $SiO_2:(Y_2O_3 + Ga_2O_3)$ einen Wert zwischen 10 und 500 hat und das Molverhältnis $Ga_2O_3:Y_2O_3$ einen Wert kleiner als 1 hat, und

b) nach einstündiger Kalzinierung an Luft bei 500°C ein Röntgenbeugungspulverdiagramm aufweist, in welchem die stärksten Linien die 4 in Tabelle A aufgeführten Linien sind

TABELLE A

| d(Å) |
| --- |
| 11.1 $\pm$ 0.2 |
| 10.0 $\pm$ 0.2 |
| 3.84 $\pm$ 0.07 |
| 3.72 $\pm$ 0.06 |

in welchem Katalysator die Menge des auf einen Träger aufgebrachten Galliums 1 bis 10 Gewichtsprozent beträgt, berechnet auf die Menge des im Katalysator vorhandenen kristallinen Metallsilikats, und welcher Katalysator mindestens einmal einer zweistufigen Behandlung unterwofen worden ist, in deren erster Stufe der Katalysator bei einer Temperatur von 350 bis 700°C mit einem Kohlenwasserstoff oder einer Kohlenwasserstoffmischung kontakiert wird, bis sich in weniger als 5 Sünden 0,1 bis 5 Gewichtsprozent Koks auf dem Katalysator niedergeschlagen haben, gefolgt von einer zweiten Stufe, in welcher der mit Koks beladene Katalysator bei einer Temperatur von 350 bis 700°C mit einem sauerstoffhaltigen Gas kontakiert wird, bis mehr als 50 Gewichtsprozent des auf dem Katalysator niedergeschlagenen Koks entfernt worden sind.

2. Ein Verfahren wie in Anspruch 1 beansprucht, dadurch gekennzeichnet, daß es auf eine Zuspeisung angewendet wird, welche zu mehr als 75 Gewichtsprozent aus 1 oder mehreren Paraffinen mit 3 oder 4 Kohlenstoffatomen im Molekül besteht.

3. Ein Verfahren wie in Anspruch 1 oder 2 beansprucht, dadurch gekennzeichnet, daß es bei einer Temperatur von 350 bis 700°C, einem Druck von 1 bis 20 bar und einer Raumgeschwindigkeit von 0,1 bis 10 kg.kg$^{-1}$.h$^{-1}$ durchgeführt wird.

4. Ein Verfahren wie in irgendeinem der Ansprüche 1 bis 3 beansprucht, dadurch gekennzeichnet, daß der Katalysator der zweistufigen Behandlung höchstens dreimal unterwofen wird.

5. Ein Verfahren wie in irgendeinem der Ansprüche 1 bis 4 beansprucht, dadurch gekennzeichnet, daß die Menge an kristallinem Metallsilikat mindestens 20 Gewichtsprozent beträgt, berechnet auf die Summe der Menge an in dem Katalysator vorhandenen kristallinen Metallsilikat und der Menge an anderen als Trägermaterial für Gallium verwendeten Materialen, welche in dem Katalysator vorliegen können.

6. Ein Verfahren wie in irgendeinem der Ansprüche 1 bis 5 beansprucht, dadurch gekennzeichnet, daß das im Katalysator vorhandene Gallium auf dem einem kristallinen Metallsilikat als Trägermaterial aufgebracht ist und daß das Gallium durch Imprägnieren oder Ionenaustausch auf dem kristallinen Metallsilikat niedergeschlagen worden ist.

7. Ein Verfahren wie in irgendeinem der Ansprüche 1 bis 6 beansprucht, dadurch gekennzeichnet, daß das im Katalysator vorhandene Gallium auf dem einem amorphen Trägermaterial aufgebracht worden ist.

8. Ein Verfahren wie in irgendeinem der Ansprüche 1 bis 7 beansprucht, dadurch gekennzeichnet, daß die Zuspeisung dazu verwendet wird, um die erforderliche Menge an Koks in der ersten Stufe des zweistufigen Behandlungsverfahrens niederzuschlagen.

9. Ein Verfahren wie in irgendeinem der Ansprüche 1 bis 8 beansprucht, dadurch gekennzeichnet, daß in der zweiten Stufe des zweistufigen Behandlungsverfahrens Luft als sauerstoffhaltiges Gas verwendet wird.

10. Ein Verfahren wie in irgendeinem der Ansprüche 1 bis 9 beansprucht, dadurch gekennzeichnet, daß in der zweiten Stufe des zweistufigen Behandlungsverfahrens mehr als 75 Gewichtsprozent des auf dem Katalysator anwesenden Koks entfernt werden.

## Revendications

1. Un procédé pour la préparation d'un mélange d'hydrocarbures aromatiques, caractérisé en ce que des paraffines présentant deux, trois ou quatre atomes de carbone par molécule ou des mélanges d'hydrocarbures aliphatiques constitués de plus de 50% en poids desdites paraffines sont mises en contact avec un catalyseur comportant du gallium supporté sur un support et un silicate métallique cristallin qui

a) a été préparé par cristallisation en partant d'une solution aqueuse qui contient un ou plus d'un composé de métal alcalin, un ou plus d'un composé organique azoté qui contient un cation organique ou à partir duquel un cation organique est formé pendant la préparation du silicaté, un ou plus d'un composé du silicium, un ou plus d'un composé d'un métal trivalent Y choisi dans le groupe constitué par l'aluminium, le fer, le cobalt et le chrome et, si désiré, un ou plus d'un composé du gallium en des quantités telles que, dans la formule qui représenté la composition du silicate exprimés en moles d'oxydes, le rapport molaire $SiO_2/(Y_2O_3 + Ga_2O_3)$ est de 10—500 et le rapport molaire $Ga_2O_3/Y_2O_3$ est inférieur à 1, et

b) aprés une calcination d'une heure à l'air à 500°C présents un diagramme de diffraction aux rayons X sur poudre dans lequel les raies les plus fortes sont les quatre raies mentionnées sur le tableau A,

TABLEAU A

| d(Å) |
|---|
| 11,1 ± 0,2 |
| 10,0 ± 0,2 |
| 3,84 ± 0,07 |
| 3,72 ± 0,06 |

catalyseur dans lequel la quantité de gallium supportée sur un support s'élève à 1—10% en poids, calculé sur la quantité de silicate cristallin métallique présent dans le catalyseur et lequel catalyseur a été soumis au moins une fois à un traitement en deux étapes, comportant une étape dans laquelle le catalyseur est mis en contact à une température de 350—700°C avec un hydrocarbure ou un mélange d'hydrocarbures jusqu'à ce que en moins de 5 heures, 0,1—5% de coke ait été déposé sur le catalyseur, suivi par une seconde étape

dans laquelle le catalyseur chargé sur coke est mis en contact à une température de 350—700°C avec un gaz renfermant de l'oxygène jusqu'à ce que plus de 50% en poids du coke présent sur le catalyseur ait été eliminé.

2. Un procédé tel que revendiqué dans la revendication 1, caractérisé en ce qu'il est appliqué à une charge dont plus de 75% en poids est constitué d'une ou des plus d'une paraffine présentant trois ou quatre atomes de carbone par molécule.

3. Un procédé tel que revendiqué dans la revendication 1 ou 2, caractérisé en ce que qu'il est réalisé à une température de 350—700°C, sous une pression de 1 à 20 bars et à une vitesse spatiale de 0,1—10 kg.kg$^{-1}$.h$^{-1}$.

4. Un procédé tel que revendiqué dans l'une quelconque des revendications 1 à 3, caractérisé en ce que le catalyseur est soumis au traitement en deux étapes au moins trois fois.

5. Un procédé tel que revendiqué dans l'une quelconque des revendications 1 à 4, caractérisé en ce que la quantité de silicate cristallin métallique, calculé sur la somme de la quantité de silicate cristallin métallique présent dans le catalyseur et la quantité d'autre matériau utilisé comme support pour le gallium qui peut être présent dans la catalyseur, est d'au moins 20% en poids.

6. Un procédé tel que revendiqué dans l'une quelconque des revendications 1 à 5, caractérisé en ce que le gallium présent dans la catalyseur est supporté sur le silicate cristallin métallique comme support et que le gallium à été déposé sur le silicate cristallin métallique par imprégnation ou échange d'ions.

7. Un procédé tel que revendiqué dans l'une quelconque des revendications 1 à 5, caractérisé en ce que le gallium présent dans le catalyseur est supporté sur un matériau support amorphe.

8. Un procédé tel que revendiqué dans l'une quelconque des revendications 1 à 7, caractérisé en ce que la charge est utilisée pour réaliser le niveau désiré de dépôt de coke dans la première étape du traitement en deux étapes.

9. Un procédé tel que revendiqué dans l'une quelconque des revendications 1 à 8, caractérisé en ce que l'air est utilisé comme gaz renfermant de l'oxygène dans la seconde étape du traitement en duex étapes.

10. Un procédé tel que revendiqué dans l'une quelconque des revendications 1 à 9, caractérisé en ce que la seconde étape du traitement en deux étapes plus de 75% en poids du coke présent sur le catalyseur est éliminé.